# FASCICULE DE BREVET EUROPEEN

(11) **EP 1 064 252 B1**
(45) Date de publication et mention de la délivrance du brevet: **15.06.2005**
(21) Numéro de dépôt: 99907710.0
(22) Date de dépôt: 15.03.1999
(51) Int. Cl.: C07C 217/84, C07D 295/08, C07D 295/14, C07C 235/60, C07D 295/12, C07C 271/28, C07C 215/76, C07C 211/63, A61K 7/13

(54) **COMPOSITION DE TEINTURE D'OXYDATION CONTENANT UN COUPLEUR CATIONIQUE, PROCEDES DE TEINTURE, NOUVEAUX COUPLEURS CATIONIQUES**
OXIDATIONSFÄRBEMITTEL MIT KATIONISCHEM KUPPLER, FÄRBEVERFAHREN, KATIONISCHE KUPPLER
OXIDATION DYEING COMPOSITIONS CONTAINING A CATIONIC COUPLING AGENT, NOVEL CATIONIC COUPLING AGENTS

(30) Priorité: 20.03.1998 FR 9803455
(43) Date de publication de la demande: 03.01.2001
(73) Titulaire: L'OREAL, 75008 Paris (FR)
(72) Inventeur: GENET, Alain, F-93600 Aulnay-sous-Bois (FR); LAGRANGE, Alain, F-77770 Coupvray (FR)
(74) Mandataire: Miszputen, Laurent
(86) Numéro de dépôt international: PCT/FR1999/000574
(87) Numéro de publication internationale: WO 1999/048856

(56) Documents cités:
- BE-A- 639 047
- FR-A- 2 520 358
- CHEMICAL ABSTRACTS, vol. 109, no. 5, 1 août 1988 Columbus, Ohio, US; abstract no. 34348, OHSAWA, SUSUMU ET AL: "Application of new synthetic substrate for estimation of serum cholinesterase activity. Measurement of pseudo-ChE activity using the new substrate (3,4-dihydroxybenzoylcholine)" XP002086280 -& Chemical Abstracts 12th Collective Index RN: 115214-68-1 Ethanaminium,-[(2,4-dihydroxybenzoyl)oxy] -N,N,N-trimethyl-, iodide XP002087716 & RINSHO KAGAKU (NIPPON RINSHO KAGAKKAI) (1987), 16(2), 106-13 CODEN: RIKAAN;ISSN: 0370-5633,1987,

## Description

L'invention a pour objet une composition pour la teinture d'oxydation des fibres kératiniques contenant au moins un coupleur monobenzénique comportant un groupement cationique Z, Z étant choisi parmi des chaînes aliphatiques quatemisées et des chaînes aliphatiques contenant au moins un cycle saturé quatemisé, l'utilisation de ces coupleurs pour la teinture des fibres kératiniques, les procédés de teinture d'oxydation les mettant en oeuvre, de nouveaux coupleurs monobenzéniques comportant au moins un groupement cationique Z, Z étant choisi parmi des chaînes aliphatiques quatemisées et des chaînes aliphatiques contenant au moins un cycle saturé quatemisé.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.
La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet FR-A-2 520 358, d'utiliser certains dérivés cationiques de méta-phénylènediamines, à savoir plus précisément certaines méta-phénylènediamines monosubstituées par une chaîne aliphatique quatemisée, pour la teinture d'oxydation des fibres kératiniques dans des nuances intenses. Toutefois, l'utilisation des métaphénylènediamines décrites dans cette demande de brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...).

Or, la demanderesse vient maintenant de découvrir, de façon totalement inattendue et surprenante, que certains composés monobenzéniques de formule (I) définie ci-après comportant un groupement cationique Z, Z étant choisi parmi des chaînes aliphatiques quatemisées et des chaînes aliphatiques contenant au moins un cycle saturé quatemisé, non seulement conviennent pour une utilisation comme coupleurs pour la coloration d'oxydation, mais en outre qu'elles permettent d'obtenir des compositions tinctoriales conduisant à des colorations puissantes, dans une très large palette de couleurs et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet une composition selon la revendication 1.

X⁻ est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate.

Comme indiqué précédemment, les colorations obtenues avec la composition de teinture d'oxydation conforme à l'invention sont puissantes et permettent d'atteindre des nuances dans une très large palette de couleurs. Elles présentent de plus d'excellentes propriétés de résistance vis à vis de l'action des différents agents extérieurs (lumière, intempéries, lavage, ondulation permanente, transpiration, frottements).

Dans la formule (I) ci-dessus les radicaux alkyle et alcoxy peuvent être linéaires ou ramifiés.

Parmi les composés de formule (I) ci-dessus, on peut notamment citer :
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de [3-(2,4-diamino-phénoxy)-propyl]-triéthyl-ammonium ;
- le chlorure de [2-(4-amino-2-hydroxy-phénoxy)-éthyl]-diéthyl-méthyl-ammonium ;
- le chlorure de 1-[3-(2-amino-4-méthylamino-phénoxy)-propyl]-1-méthyl-pipéridinium ;
- le chlorure de [2-(2,4-diamino-phénoxy)-éthyl]-diéthyl-méthyl-ammonium ;
- le chlorure de 1-[3-(2,4-diamino-phénoxy)-propyl]-1-méthyl-pyrrolidinium ;
et leurs sels d'addition avec un acide.

Parmi ces composés de formule (I), on préfère plus particulièrement:
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
et leurs sels d'addition avec un acide.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Selon une forme de réalisation préférée de l'invention, la composition tinctoriale renferme en outre une ou plusieurs bases d'oxydation qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphényiènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénytènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) -éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines différentes des méta-phénylènediamines de formule (I), les méta-aminophénols différents des méta-aminophénols de formule (I), les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanotamines telles que les mono-, di- et triéthanolamines ainsi que leurs dérivés, les hydroxydes de sodium ou de potassium et les composés de formule (III) suivante : 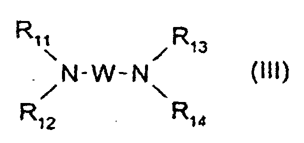 dans laquelle W est un reste propylène éventuellement substitué par un groupement hydroxyle ou un radical alkyle en C₁-C₆ ; R₁₁, R₁₂, R₁₃ et R₁₄, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases et les oxydo-réductases à 2 électrons. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains composés de formule (I) sont nouveaux en soi et constituent à ce titre un autre objet de l'invention. Ces nouveaux composés, ainsi que leur sels d'addition avec un acide répondent à la formule (I') selon la revendication 19.

Parmi les composés de formule (I') ci-dessus, on peut notamment citer:
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de [3-(2,4-diamino-phénoxy)-propyl]-triéthyl-ammonium ;
- le chlorure de [2-(4-amino-2-hydroxy-phénoxy)-éthyl]-diéthyl-méthyl-ammonium ;
- le chlorure de 1-[3-(2-amino-4-méihylamino-phénoxy)-propyl]-1-méthyl-pipéridinium ;
- le chlorure de [2-(2,4-diamino-phénoxy)-éthyl]-diéthyl-méthyl-ammonium ;
- le chlorure de 1-[3-(2,4-diamino-phénoxy)-propyl]-1-méthyl-pyrrolidinium ;
et leurs sels d'addition avec un acide.

Les composés de formule (I') conformes à l'invention peuvent être facilement obtenus, selon des méthodes bien connues de l'état de la technique par exemple par réduction des composés nitrés cationiques correspondants (méta-nitranilines cationiques ou méta-nitrophénols cationiques),

Cette étape de réduction (obtention d'une amine aromatique primaire) suivie ou non d'une salification, est en général, par commodité, la dernière étape de la synthèse.

Cette réduction peut intervenir plus tôt dans la suite des réactions conduisant à la préparation des composés de formule (I'), et selon des procédés bien connus il faut alors "protéger" l'amine primaire créée (par exemple par une étape d'acétylation, de benzènesulfonation, etc...), faire ensuite la ou les substitutions ou modifications désirées (y compris la quatemisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I') conformes à l'invention peuvent, le cas échéant, être récupérés par des méthodes bien connues de l'état de la technique telles que la cristallisation ou la distillation.

Un autre objet de l'invention est l'utilisation des composés de formule (I) ou de formule (I') conformes à l'invention à titre de coupleur pour la teinture d'oxydation des fibres kératiniques, et en particulier des fibres kératiniques humaines telles que les cheveux.

Les exemples qui suivent sont destinés à illustrer l'invention sans pour autant en limiter la portée.

### EXEMPLES DE PREPARATION

### EXEMPLE 1 : Préparation du chlorure de [2-(2,4-diamino-phénoxy)-éthyl]-diéthyl-méthyl-ammonium ; dichlorhydrate

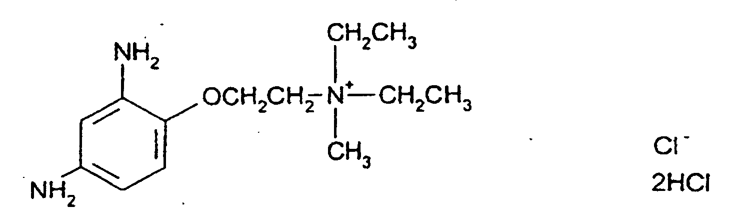

### a) Synthèse du N-[2-(2-diéthylamino-éthoxy)-5-nitro-phényl]-acétamide

On a chauffé au bain-marie bouillant la suspension de 35,1 g (0,15 mole) de sel de potassium du N-(2-hydroxy-5-nitro-phényl)-acétamide et de 23,0 g (0,17 mole) de (2-chloro-éthyl)-diéthyl-amine dans 105 ml de diméthylformamide. On a versé dans 450 ml d'eau glacée légèrement sodique, essoré le précipité cristallisé, réempaté dans l'eau et séché sous vide, à 45°C, sur anhydride phosphorique.
Après recristallisation du méthanol au reflux on a obtenu des cristaux jaune pâle (22,8 g) de N-[2-(2-diéthylamino-éthoxy)-5-nitro-phényl]-acétamide qui ont fondu à 116°C et dont l'analyse élémentaire calculée pour C₁₄H₂₁N₃O₄ était :

| % | C | H | N |
|---|---|---|---|
| Calculé | 56,94 | 7,17 | 14,23 |
| Trouvé | 57,15 | 7,47 | 14,35 |

### b) Synthèse du iodure de [2-(2,4-bis-acétylamino-phénoxy)-éthyl]-diéthyl-méthyl-ammonium

Dans un hydrogénateur on a placé 15,0 g (0,05 mole) de N-[2-(2-diéthylamino-éthoxy)-5-nitro-phényl]-acétamide obtenu ci-dessus à l'étape précédente, 1 g de palladium à 5% sur charbon (contenant 50% d'eau) et 60 ml d'éthanol.
La réduction s'est faite en une ½ heure sous une pression d'hydrogène d'environ 8 bars et à une température qui a progressivement été portée à 80°C. Après filtration du catalyseur sous azote on a évaporé à sec sous pression réduite, repris dans 45 ml de dioxane et acétylé avec 6 g d'anhydride acétique. Le précipité blanc a été essoré, lavé dans le dioxane et séché sous vide à 50°C. On a obtenu 15,3 g de cristaux blancs de N-[5-acétylamino-2-(2-diéthylamino-éthoxy)-phényl]-acétamide qui ont été dissous dans 150 ml d'acétone au reflux. On a quaternisé en ajoutant goutte à goutte 10,6 g (0,075mole) d'iodure de méthyle.
On a agité pendant 10 minutes, essoré le précipité blanc obtenu, lavé à l'acétone bouillant et séché sous vide à 45°C.
On a obtenu 21,0 g de cristaux blancs de iodure de [2-(2,4-bis-acétylamino-phénoxy)-éthyl]-diéthyl-méthyl-ammonium dont l'analyse élémentaire calculée pour C₁₇H₂₈N₃O₃I était :

| % | C | H | N O | | I |
|---|---|---|---|---|---|
| Calculé | 45,44 | 6,28 | 9,35 | 10,68 | 28,24 |
| Trouvé | 45,34 | 6,25 | 9,32 | 10,83 | 28,28 |

### c) Désacétylation du iodure de [2-(2,4-bis-acétylamino-phénoxy)-éthyl]-diéthyl-méthyl-ammonium

On a chauffé une heure au reflux 21,0 g (0,0467 mole) de iodure de [2-(2,4-bis-acétylamino-phénoxy)-éthyl]-diéthyl-méthyl-ammonium obtenu ci-dessus à l'étape précédente dans 35 ml d'acide chlorhydrique 36% et 35 ml d'éthanol.
La solution a été refroidie et diluée avec 100 ml d'éthanol.
Le précipité blanc a été essoré, lavé à l'acétone et séché sous vide à 50°C sur potasse.
On a obtenu 11,7 g de cristaux blancs de chlorure de [2-(2,4-diamino-phénoxy)-éthyl]-diéthyl-méthyl-ammonium, dichlorhydrate dont l'analyse élémentaire calculée pour C₁₃H₂₆N₃OCl₃ était :

| % | C | H | N | O | Cl |
|---|---|---|---|---|---|
| Calculé | 45,03 | 7,56 | 12,12 | 4,61 | 30,67 |
| Trouvé | 44,86 | 7,51 | 12,03 | 4,70 | 30,83 |

### EXEMPLE 2 : Préparation du chlorure de 1-[3-(2,4-diamino-phénoxy)-propyl]-1,4-diméthyl-pipérazin-1-ium, trichlorhydrate, dihydrate

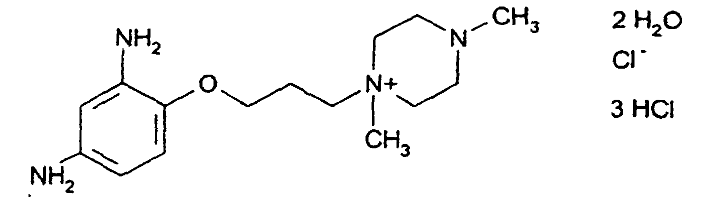

### a) Synthèse du N-[2-(3-chloro-propoxy)-5-nitro-phényl]-acétamide

A la suspension de 294,2 g (1,5 mole) de N-(2-hydroxy-5-nitro-phényl)-acétamide et de 228,0 g (1,65 mole) de carbonate de potassium dans 900 ml de diméthylformamide agitée à température ambiante, on a ajouté en 50 minutes 708,5 g (4,5 moles) de 1-bromo-3-chloro-propane.
La suspension orangée a été agitée pendant 4 heures à température ambiante puis pendant 2 heures à 28-30°C.
On a versé le mélange réactionnel dans 4 litres d'eau glacée ; une huile a précipité.
Cette huile a été décantée, reprise dans 500 ml d'isopropanol ; les cristaux obtenus ont été essorés et recristallisés de l'isobutanol au reflux.
On a obtenu des cristaux jaune pâle (84,4 g) de N-[2-(3-chtoro-propoxy)-5-nitro-phényl]-acétamide qui ont fondu à 130°C (Kofler) et dont l'analyse élémentaire calculée pour C₁₁H₁₃N₂O₄Cl était :

| % | C | H | N | O | Cl |
|---|---|---|---|---|---|
| Calculé | 48,45 | 4,81 | 10,27 | 23,47 | 13,00 |
| Trouvé | 48,05 | 4,82 | 10,16 | 23,33 | 12,60 |

### b) Synthèse du chlorure de 1-[3-(2-acétylamino-4-nitro-phénoxy)-propyl]-1,4-diméthyl-pipérazin-1-ium

On a chauffé au reflux de l'isobutanol (300 ml) pendant 25 heures la solution de 81,8 g (0,3 mole) de N-[2-(3-chloro-propoxy)-5-nitro-phényl]-acétamide obtenu ci-dessus à l'étape précédente et de 62,8 g (0,55 mole) de 1,4-diméthyl-pipérazine.
Le composé quatemisé cristallisé a précipité ; il a été essoré, lavé à l'éthanol absolu et recristallisé de l'éthanol 96 au reflux.

On a obtenu des cristaux jaune clair (55,0 g) de chlorure de 1-[3-(2-acétylamino-4-nitro-phénoxy)-propyl]-1,4-diméthyl-pipérazin-1-ium qui ont fondu à 214°C (Kofler) et dont l'analyse élémentaire calculée pour C₁₇H₂₇N₄O₄Cl + ½ H₂O était :

| % | C | H | N | O | Cl |
|---|---|---|---|---|---|
| Calculé | 51,58 | 7,13 | 14,15 | 18,19 | 8,96 |
| Trouvé | 51,46 | 7,05 | 13,88 | 17,44 | 8,80 |

### c) Synthèse du chlorure de 1-[3-(2-acétylamino-4-amino-phénoxy)-propyl]-1,4-diméthyl-pipérazin-1-ium

Dans un hydrogénateur on a placé 55,0 g (0,142 mole) de chlorure de 1-[3-(2-acétylamino-4-nitro-phénoxy)-propyl]-1,4-diméthyl-pipérazin-1-ium obtenu ci-dessus à l'étape précédente, 10 g de palladium à 5% sur charbon (contenant 50% d'eau), 300 ml d'eau et 300 ml d'isopropanol.
La réduction s'est faite en une ½ heure sous une pression d'hydrogène d'environ 9 bars et à une température qui a progressivement été portée à 70°C.
Après filtration du catalyseur sous azote, on a évaporé à sec sous pression réduite (cristaux).
On a recristallisé de l'éthanol à 96° au reflux et obtenu des cristaux blancs de chlorure de 1-[3-(2-acétylamino-4-amino-phénoxy)-propyl]-1,4-diméthyl-pipérazin-1-ium (28,0 g) qui ont fondu à 226°C (Kofler) et dont l'analyse élémentaire calculée pour C₁₇H₂₉N₄O₂Cl + ½ H₂O était :

| % | C | H | N | O | Cl |
|---|---|---|---|---|---|
| Calculé | 55,80 | 8,26 | 15,31 | 10,93 | 9,69 |
| Trouvé | 55,84 | 8,08 | 15,26 | 10,28 | 9,63 |

### d) Désacétylation du chlorure de 1-[3-(2-acétylamino-4-amino-phénoxy)-propyl]-1,4-diméthyl-pipérazin-1-ium

On a chauffé pendant deux heures au reflux 28,0 g (0,0784 mole) de chlorure de 1-[3-(2-acétylamino-4-amino-phénoxy)-propyl]-1,4-diméthyl-pipérazin-1-ium obtenu ci-dessus à l'étape précédente dans 50 ml d'acide chlorhydrique 36% et 100 ml d'éthanol.
La solution a été refroidie ; la gomme précipitée a été séparée et reprise dans l'éthanol absolu (cristallisation).
On a essoré et recristallisé d'un mélange éthanol/acide chlorhydrique 36% au reflux.
On a obtenu des cristaux blancs de chlorure de 1-[3-(2,4-diamino-phénoxy)-propyl]-1,4-diméthyl-pipérazin-1-ium, trichlorhydrate, dihydrate (24,9 g) qui ont fondu avec décomposition à 255-260°C (Kofler) dont la structure en RMN 1H était conforme au produit attendu et dont l'analyse élémentaire calculée pour C₁₅H₃₀N₄OCl₄ + 2H₂O était :

| % | C | H | N | O | Cl |
|---|---|---|---|---|---|
| Calculé | 39,14 | 7,85 | 12,17 | 10,43 | 30,81 |
| Trouvé | 39,02 | 7,56 | 11,85 | 10,72 | 30,45 |

### EXEMPLES D'APPLICATION

### EXEMPLES 1 à 3 DE TEINTURE EN MILIEU BASIQUE

On a préparé les compositions tinctoriales suivantes (teneurs en grammes) :

| **EXEMPLE** | **1** | **2** | **3** |
|---|---|---|---|
| Chlorure de [2-(2,4-diamino-phénoxy)-éthyl]-diéthyl-méthyl-ammonium ; dichlorhydrate (composé de formule (I)) | 1,04 | 0,693 | 0,693 |
| Para-aminophénol (Base d'oxydation) | 0,327 | - | - |
| Paraphénylènediamine (Base d'oxydation) | - | 0,216 | - |
| Dichlorhydrate de 4,5-diamino-1-éthyl-3-méthyl pyrazole (Base d'oxydation) | - | - | 0,426 |
| Support de teinture commun n°1 | (*) | (*) | (*) |
| Eau déminéralisée q.s.p. | 100 g | 100 g | 100 g |

| | | | |
|---|---|---|---|
| (*) Support de teinture commun n° 1 : | | | |

- Alcool benzylique 2 g
- Polyéthylène glycol à 6 moles d'oxyde d'éthylène 3 g
- Ethanol à 96° 18 g
- Alkyl (C₈-C₁₀) polyglucoside en solution aqueuse à 60 % de matière active (M.A.) tamponné par du citrate d'ammonium, vendu sous la dénomination ORAMIX CG110 ® par la société SEPPIC 6 g
- Ammoniaque à 20 % de NH₃ 10 g
- Métabisuifite de sodium 0,23 g
- Agent séquestrant q.s.

Au moment de l'emploi, on a mélangé la composition tinctoriale de l'exemple 1 ci-dessus poids pour poids avec une solution de peroxyde d'hydrogène à 20 volumes (6 % en poids) de pH 3.
Au moment de l'emploi, on a mélangé chacune des compositions tinctoriales des exemples 2 et 3 ci-dessus poids pour poids avec une composition oxydante constituée par une solution aqueuse à 6.10⁻³ mol% de persulfate d'ammonium.

Chacun des mélanges obtenus a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de TEINTURE** | **Nuance sur cheveux naturels** | **Nuance sur cheveux permanentés** |
|---|---|---|---|
| 1 | 10 ± 0,2 | Irisé légèrement cendré | Irisé violacé |
| 2 | 10± 0,2 | Bleu cendré | Bleu profond |
| 3 | 10 ± 0,2 | Violet cendré | Violet intense |

### EXEMPLE 4 DE TEINTURE EN MILIEU ACIDE

On a préparé la composition tinctoriale suivante :
- Chlorure de [2-(2,4-diamino-phénoxy)-éthyl]-diéthyl-méthyl-ammonium dichlorhydrate (composé de formule (I)) 0,693 g
- Paratoluylènediamine (Base d'oxydation) 0,244 g
- Alcool benzylique 2 g
- polyéthylène glycol à 6 moles d'oxyde d'éthylène 3 g
- Ethanol à 96° 18 g
- Alkyl(C₈-C₁₀)polyglucoside en solution aqueuse à 60 % de matière active (M.A.) tamponné par du citrate d'ammonium, vendu sous la dénomination ORAMIX CG110 ® par la société SEPPIC 6 g
- Tampon K₂HPO₄/KH₂PO₄ (1,5 M / 1 M) 10 g
- Métabisulfite de sodium 0,23 g
- Agent séquestrant q.s.
- Eau déminéralisée q.s.p. 100 g

Au moment de l'emploi, la composition tinctoriale ci-dessus a été mélangée poids pour poids avec une solution de peroxyde d'hydrogène à 20 volumes (6% en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de TEINTURE** | **Nuance sur cheveux naturels** | **Nuance sur cheveux permanentés** |
|---|---|---|---|
| 4 | 5.7 ± 0,2 | Cendré bleu | Cendré bleu puissant |

### EXEMPLE 5 DE COLORATION ENZYMATIQUE

On a préparé la composition tinctoriale suivante :
- Chlorure de [2-(2,4-diamino-phénoxy)-éthyl]-diéthyl-méthyl-ammonium dichlorhydrate (composé de formule (I)) 1,04 g
- Paraphénylènediamine (Base d'oxydation) 0,324 g.
- Monométhyléther de propylène glycol 10 g
- Copolymère acide acrytique / acrylate d'alkyle (C₁₀ / C₃₀) réticulé 0,25 g
- Alcool taurique oxyéthyléné à 12 moles d'oxyde d'éthylène 2 g
- Acide urique 1 g
- Uricase d'Arthrobacter globiformis à 20 Unités Internationales (U.I.) / mg commercialisée par la Société SIGMA 1 g
- Agent antioxydant 0,1 g
- 2-amino-2-méthyl-1-propanol q.s.p. pH 9,5
- Eau déminéralisée q.s.p. 100 g

La composition tinctoriale ci-dessus a été appliquée pendant 30 minutes sur des éprouvettes de 20 mg de cheveux gris, naturels ou permanentés, à 90 % de blancs. Les cheveux ont ensuite été rincés, lavés au shampooing, rincés à nouveau puis séchés.

Les nuances obtenues figurent dans le tableau ci-après

| **EXEMPLE** | **Nuance sur cheveux naturels** | **Nuance sur cheveux permanentés** |
|---|---|---|
| 5 | Bleu puissant légèrement cendré | Bleu puissant légèrement cendré |

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un coupleur monobenzénique de formule (I) suivante, et/ou au moins un de leurs sels d'addition avec un acide : 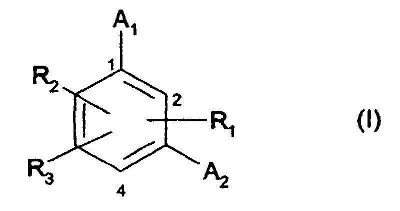 dans laquelle:
• R₁, R₂, R₃, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosuifonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ; ou un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-alkyl(C₁-C₆)aminosulfonyle, N,N-dialkyl(C₁-C₆)aminosulfonyle, thiocarbamyle, formyte ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylatkyle en C₁-C₆ ; un radical N,N-dialkyl(C₁-C₆)carbamytalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆; un radical alkyl(C₁-C₆)sulfonylalkyle en C₁-C₆ ; un radical alkyl(C₁-C₆)carbonylalkyle en C₁-C₆ ;un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
• A1 représente un groupement -NR₄R₅ ou un radical hydroxyle;
• A2 représente un groupement -NR'₄R'₅ ou un radical hydroxyle ;
• R₄, R'₄, R₅ et R'₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
• Z représente un groupement de formule (II) suivante : 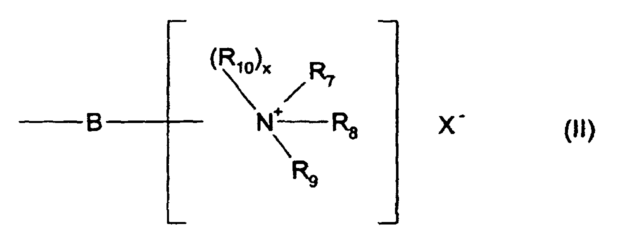 dans laquelle:
• B est un bras de liaison qui représente une chaîne alkyle comportant de préférence de 1 à 14 atomes de carbone, linéaire ou ramifiée;
• R₇, R₈ et R₉, identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical cyanoalkyle en C₁-C₆ ; un radical aryle ; un radical benzyle ; un radical carbamylalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyl en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
• X⁻ représente un anion monovalent ou divalent ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est un nombre entier égal à 0 ou 1 ; avec les conditions suivantes :
- lorsque x = 0, alors le bras de liaison B est rattaché à l'atome d'azote portant les radicaux R₇ à R₉ ;
- lorsque x = 1, alors deux des radicaux R₇ à R₉ forment, conjointement avec l'atome d'azote auquel ils sont rattachés, un cycle saturé à 5 ou 6 chaînons tel que défini précédemment, et le bras de liaison B est porté par un atome de carbone dudit cycle saturé ;
étant entendu que le nombre de groupements Z est égal à 1.

2. Composition selon la revendication 1, **caractérisée par le fait que** X⁻ représente un atome d'halogène tel que le chlore, le brome, le fluor ou l'iode, un hydroxyde, un hydrogènesulfate, ou un alkyl(C₁-C₆)sulfate.

3. Composition selon la revendication 1 ou 2, **caractérisée par le fait que** les composés de formule (I) sont choisis parmi :
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de [3-(2,4-diamino-phénoxy)-propyl]-triéthyl-ammonium ;
- le chlorure de [2-(4-amino-2-hydroxy-phénoxy)-éthyl)-diéthyl-méthyl-ammonium ;
- le chlorure de 1-[3-(2-amino-4-méthylamino-phénoxy)-propyl]-1-methyl-pipéridinium ;
- le chlorure de [2-(2,4-diamino-phénoxy)-éthyl]-diéthyl-méthyl-ammonium ;
- le chlorure de 1-[3-(2,4-diamino-phénoxy)-propyl]-1-méthyl-pyrrolidinlum ;
et leurs sels d'addition avec un acide.

4. Composition selon la revendication 3, **caractérisée par le fait que** les composés de formule (I) sont choisis parmi :
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
et leurs sels d'addition avec un acide.

5. Composition selon l'une quelconque des revendications précédentes, **caractérisée par le fait que** le ou les composés de formule (I) et/ou le ou leurs sels d'addition avec un acide représentent de 0,0005 à 12 % en poids du poids total de la composition tinctoriale.

6. Composition selon la revendication 5, **caractérisée par le fait que** le ou les composés de formule (I) et/ou le ou leurs sels d'addition avec un acide représentent de 0,005 à 6 % en poids du poids total de la composition tinctoriale.

7. Composition selon l'une quelconque des revendications précédentes, **caractérisée par le fait qu'**elle renferme au moins une base d'oxydation choisie parmi les paraphénylènediamines, les bis-phénylalkylènediamines, les para-aminophénols, les ortho-aminophénols et les bases hétérocycliques.

8. Composition selon la revendication 7, **caractérisée par le fait que** la ou les bases d'oxydation représentent de 0,0005 à 12% en poids du poids total de la composition tinctoriale.

9. Composition selon la revendication 8, **caractérisée par le fait que** la ou les bases d'oxydation représentent de 0,005 à 6% en poids du poids total de la composition tinctoriale.

10. Composition selon l'une quelconque des revendications précédentes, **caractérisée par le fait qu'**elle renferme un ou plusieurs coupleurs additionnels choisis parmi les métaphénylènediamines, les méta-aminophénols, les métadiphénols et les coupleurs hétérocycliques.

11. Composition selon la revendication 10, **caractérisée par le fait que** le ou les coupleurs additionnels représentent de 0,0001 à 10 % en poids du poids total de la composition tinctoriale.

12. Composition selon la revendication 11, **caractérisée par le fait que** le ou les coupleurs additionnels représentent de 0,005 à 5 % en poids du poids total de la composition tinctoriale.

13. Composition selon l'une quelconque des revendications précédentes, **caractérisée par le fait que** les sels d'addition avec un acide sont choisis parmi les chlorhydrates, les bromhydrates, les sulfates, les citrates, les succinates, les tartrates, les lactates et les acétates.

14. Composition selon l'une quelconque des revendications précédentes, **caractérisée par le fait que** le milieu approprié pour la teinture (ou support) est constitué par de l'eau ou par un mélange d'eau et d'au moins un solvant organique.

15. Composition selon l'une quelconque des revendications précédentes, **caractérisée par le fait qu'**elle présente un pH compris entre 3 et 12.

16. Procédé de teinture d'oxydation des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

17. Procédé selon la revendication 16, **caractérisé par le fait que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à 2 électrons.

18. Dispositif à plusieurs compartiments, ou "kit" de teinture à plusieurs compartiments, dont un premier compartiment renferme une composition tinctoriale telle que définie à l'une quelconque des revendications 1 à 15 et un second compartiment renferme une composition oxydante.

19. Composés de formule (I'), et leurs sels d'addition avec un acide : 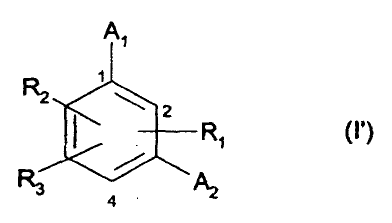 dans laquelle :
• R₁, R₂, R₃, qui peuvent être identiques ou différents, représentent un atome d'hydrogène ; un atome d'halogène ; un radical alkyl(C₁-C₆) carbonyle ; un radical aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy ; un radical alkyl(C₁-C₆) sulfonyle ; un radical aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-alkyl(C₁-C₆)aminosulfonylalkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ; ou un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆) carboxy, carbamyle, N-alkyl(C₁-C₆)carbamyle, N,N-dialkyl(C₁-C₆)carbamyle, alkyl(C₁-C₆)sulfonyle, aminosulfonyle, N-alkyl(C₁-C₆)aminosulfonyle, N,N-dialkyl(C₁-C₆)aminosulfonyle, thiocarbamyle, formyle ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ;un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle ;
• A1 représente un groupement -NR₄R₅ ou un radical hydroxyle;
• A2 représente un groupement -NR'₄R'₅ ou un radical hydroxyle;
• R₄, R'₄, R₅ et R'₅, identiques ou différents, représentent un atome d'hydrogène ; un radical alkyle en C₁-C₆ ; un radical monohydroxyalkyle en C₁-C₆ ; un radical polyhydroxyalkyle en C₂₇-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
• Z représente un groupement de formule (II) suivante : 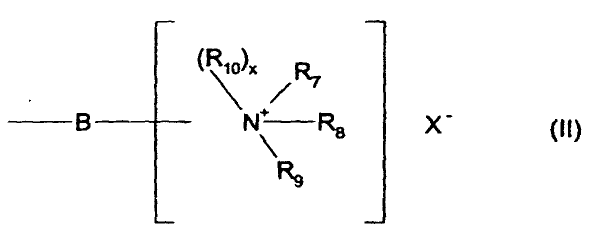 dans laquelle :
• B est un bras de liaison qui représente une chaîne alkyle comportant de préférence de 1 à 14 atomes de carbone, linéaire ou ramifiée ;
• R₇, R₈ et R₉, identiques ou différents, représentent un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical cyanoalkyle en C₁-C₆ ; un radical aryle ; un radical benzyle ; un radical carbamylalkyle en C₁-C₆ ; un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyl en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
• X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsutfate ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
• x est un nombre entier égal à 0 ou 1 ; avec les conditions suivantes :
- lorsque x = 0, alors le bras de liaison B est rattaché à l'atome d'azote portant les radicaux R₇ à R₉ ;
- lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
étant entendu que le nombre de groupements Z est égal à 1.

20. Composés selon la revendication 19, **caractérisés par le fait qu'**ils sont choisis parmi :
- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de [3-(2,4-diamino-phénoxy)-propyl]-triéthyl-ammonium ;
- le chlorure de [2-(4-amino-2-hydroxy-phénoxy)-éthyl]-diéthyl-méthyl-ammonium ;
- le chlorure de 1-[3-(2-amino-4-méthylamino-phénoxy)-propyl]-1-méthyl-pipéridinium ;
- le chlorure de [2-(2,4-diamino-phénoxy)-éthyl]-diéthyl-méthyl-ammonium ;
- le chlorure de 1-[3-(2,4-diamino-phénoxy)-propyl]-1-méthyl-pyrrolidinium ;
et leurs sels d'addition avec un acide.

21. Utilisation des composés de formule (I) tels que définis à l'une quelconque des revendications 1 à 4, à titre de coupleur pour la teinture d'oxydation des fibres kératiniques, et en particulier des fibres kératiniques humaines telles que les cheveux.

## Patentansprüche

1. Zusammensetzung zum oxidativen Färben von Keratinfasern und insbesondere menschlichen Keratinfasern, wie Haaren, **dadurch gekennzeichnet, dass** sie in einem zum Färben geeigneten Medium mindestens einen Kuppler auf Monobenzolbasis der folgenden Formel (I) und/oder mindestens ein Additionssalz dieser Verbindungen mit einer Säure enthält: 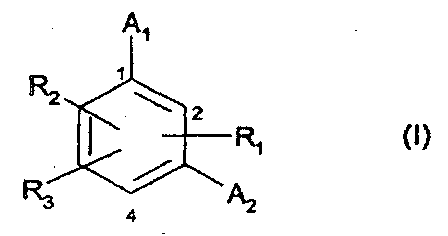 worin bedeuten:
· die Gruppen R₁, R₂ und R₃, die identisch oder voneinander verschieden sein können, ein Wasserstoffatom; ein Halogenatom; Alkyl(C₁₋₆)carbonyl; Aminoalkyl(C₁₋₆)carbonyl; N-Alkyl(C₁₋₆)-aminoalkyl(C₁₋₆)carbonyl; N,N-Dialkyl(C₁₋₆)aminoalkyl(C₁₋₆)-carbonyl; Aminoalkyl(C₁₋₆)carbonylalkyl(C₁₋₆); N-Alkyl(C₁₋₆)-aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Alkyl(C₁₋₆)aminosulfonyl; N,N-Dialkyl(C₁₋₆)aminosulfonyl; Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; C₁₋₆-Monohydroxyalkyl; C₂₋₆-Polyhydroxyalkyl; Alkoxy(C₁₋₆)-alkyl(C₁₋₆); C₁₋₆-Trifluoralkyl; Cyano; OR₆; oder eine Aminogruppe, die mit Alkyl(C₁₋₆)carbonyl, Alkyl(C₁₋₆)carboxy, Trifluoralkyl(C₁₋₆)carbonyl, Aminoalkyl(C₁₋₆)carbonyl N-Alkyl(C₁₋₆)aminoalkyl(C₁₋₆)carbonyl, N,N-Dialkyl(C₁₋₆)aminoalkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Alkyl(C₁₋₆)sulfonyl, Aminosulfonyl, N-Alkyl(C₁₋₆)aminosulfonyl, N,N-Dialkyl(C₁₋₆)aminosulfonyl, Thiocarbamoyl oder Formyl geschützt ist;
· R₆ C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); Aryl; Benzyl; Carboxyalkyl-(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)-carbamoylalkyl(C₁₋₆); C₁₋₆-Trifluoralkyl; Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); Aminoalkyl(C₁₋₆); C₁₋₆-Aminoalkyl, wobei die Aminogruppe mit einer oder zwei Gruppen substituiert ist, die gleich oder verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Thiocarbamoyl, Alkyl(C₁₋₆)sulfonyl;
· A1 -NR₄R₅ oder Hydroxy;
· A2 -NR'₄R'₅ oder Hydroxy;
· die Gruppen R₄, R'₄, R₅ und R'₅, die gleich oder verschieden sind, Wasserstoff; C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl; Thiocarbamoylalkyl(C₁₋₆); C₁₋₆-Trifluoralkyl; C₁₋₆-Sulfoalkyl; Alkyl(C₁₋₆)carboxyalkyl(C₁₋₆); Alkyl-(C₁₋₆)sulfinylalkyl(C₁₋₆); Aminosulfonylalkyl(C₁₋₆); N-Alkyl(C₁₋₆)-aminosulfonylalkyl(C₁₋₆); N,N-Dialkyl(C₁₋₆)aminosulfonylalkyl(C₁₋ ₆); Alkyl(C₁₋₆)carbonylalkyl(C₁₋₆); Aminoalkyl(C₁₋₆); C₁₋₆-Aminoalkyl, wobei die Aminogruppe mit einer oder zwei gleichen oder verschiedenen Gruppen substituiert ist, die unter Alkyl, C₁₋₆-Monohydroxyalkyl, C₂₋₆-Polyhydroxyalkyl, Alkyl(C₁₋₆)carbonyl, Carbamoyl, N-Alkyl(C₁₋₆)carbamoyl oder N,N-Dialkyl(C₁₋₆)-carbamoyl, Alkyl(C₁₋₆)sulfonyl, Formyl, Trifluoralkyl(C₁₋₆)-carbonyl, Alkyl(C₁₋₆)carboxy oder Thiocarbamoyl ausgewählt sind;
Z eine Gruppe der folgenden Formel (II) 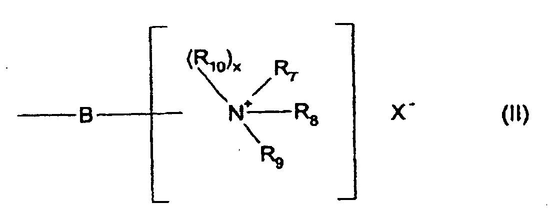 worin bedeuten:
- B eine Verbindungsgruppe, die eine Alkylkette bedeutet, welche vorzugsweise 1 bis 14 Kohlenstoffatome aufweist und die geradkettig oder verzweigt vorliegt;
- die Gruppen R₇, R₈ und R₉, die gleich oder verschieden sind, C₁₋₆-Alkyl; C₁₋₆-Monohydroxyalkyl; C₂₋₆-Polyhydroxyalkyl; Alkoxy(C₁₋₆)alkyl(C₁₋₆); Cyanoalkyl(C₁₋₆); Aryl; Benzyl; Carbamoylalkyl(C₁₋₆); Trialkyl(C₁₋₆)silanalkyl(C₁₋₆) oder eine C₁₋₆-Aminogruppe, deren Aminogruppe mit Alkyl(C₁₋₆)carbonyl, Carbamoyl oder Alkyl(C₁₋₆)sulfonyl geschützt ist; wobei zwei der Gruppen R₇, R₈ und R₉ gemeinsam mit dem Stickstoffatom, an das sie gebunden sind, einen 5- oder 6-gliedrigen gesättigten Ring auf Kohlenstoffbasis oder Ring mit einem oder mehreren Heteroatomen bilden können, wobei der Ring gegebenenfalls substituiert ist mit Halogen, Hydroxy, C₁₋₆-Alkyl, C₁₋₆-Monohydroxyalkyl, C₂₋₆-Polyhydroxyalkyl, Nitro, Cyano, Cyanoalkyl(C₁₋₆), C₁₋₆-Alkoxy, Trialkyl(C₁₋₆)silanalkyl(C₁₋₆), Amido, Aldehydo, Carboxy, Alkylcarbonyl(C₁₋₆), Thio, C₁₋₆-Thioalkyl, Alkyl(C₁₋₆)thio, Amino, einer Aminogruppe, die mit Alkyl(C₁₋₆)carbonyl, Carbamoyl oder Alkyl(C₁₋₆)-sulfonyl geschützt ist;
- X⁻ ein einwertiges oder zweiwertiges Anion;
- R₁₀C₁₋₆-Alkyl; C₁₋₆-Monohydroxyalkyl; C₂₋₆-Polyhydroxyalkyl; Aryl; Benzyl; Aminoalkyl(C₁₋₆), oder eine C₁₋₆-Aminoalkylgruppe, deren Aminogruppe mit Alkyl(C₁₋₆)carbonyl, Carbamoyl oder Alkyl(C₁₋₆)sulfonyl geschützt ist; Carboxyalkyl(C₁₋₆); Cyanoalkyl(C₁₋₆); Carbamoylalkyl(C₁₋₆); C₁₋₆-Trifluoralkyl; Trialkyl(C₁₋₆)silanalkyl(C₁₋₆); Sulfonamidoalkyl(C₁₋₆); Alkyl(C₁₋₆)-carboxyalkyl(C₁₋₆); Alkyl(C₁₋₆)sulfinylalkyl(C₁₋₆); Alkyl(C₁₋₆)-sulfonylalkyl(C₁₋₆); Alkyl(C₁₋₆)ketoalkyl(C₁₋₆); N-Alkyl(C₁₋₆)-carbamoylalkyl; N-Alkyl(C₁₋₆)sulfonamidosulfonylalkyl(C₁₋₆);
- x 0 oder 1, mit den folgenden Maßgaben:
- wenn x = 0, ist die Verbindungsgruppe B an das Stickstoffatom gebunden, das die Gruppen R₇ bis R₉ trägt,
- wenn x = 1, bilden zwei der Gruppen R₇ bis R₉ gemeinsam mit dem Stickstoffatom, an das sie gebunden sind, einen oben definierten gesättigten, 5- oder 6-gliedrigen Ring und die Verbindungsgruppe B wird von einem Kohlenstoffatom des gesättigten Rings getragen;
mit der Maßgabe, dass die Anzahl der Gruppen Z 1 beträgt.

2. Zusammensetzung nach Anspruch 1, **dadurch gekennzeichnet, dass** X- ein Halogenatom, wie Chlor, Brom, Fluor oder Iod, Hydroxid, Hydrogensulfat oder Alkyl(C₁₋₆)sulfat bedeutet.

3. Zusammensetzung nach Anspruch 1 oder 2, **dadurch gekennzeichnet, dass** die Verbindungen der Formel (I) ausgewählt sind unter:
- [2-(2,4-Diaminophenoxy)-ethyl]-diethyl-methylammoniumchlorid;
- 1-[3-(2,4-Diaminophenoxy)-propyl]-1,4-dimethyl-piperazin-1-ium-chlorid;
- [3-(2,4-Diaminophenoxy)-propyl]-triethyl-ammoniumchlorid;
- [2-(4-Amino-2-hydroxy-phenoxy)-ethyl]-diethyl-methylammoniumchlorid;
- 1-[3-(2-Amino-4-methylamino-phenoxy)-propyl]-1-methylpiperidiniumchlorid;
- [2-(2,4-Diaminophenoxy)-ethyl]-diethyl-methyl-ammoniumchlorid;
- 1-[3-(2,4-Diaminophenoxy)-propyl]-1-methyl-pyrrolidiniumchlorid;
- und deren Additionssalzen mit einer Säure.

4. Zusammensetzung nach Anspruch 3, **dadurch gekennzeichnet, dass** die Verbindungen der Formel (I) ausgewählt sind unter:
- [2-(2,4-Diaminophenoxy)-ethyl]-diethyl-methylammoniumchlorid;
- 1-[3-(2,4-Diaminophenoxy)-propyl]-1,4-dimethyl-piperazin-1-iumchlorid;
- und deren Additionssalzen mit einer Säure.

5. Zusammensetzung nach einem der vorhergehenden Ansprüche, **dadurch gekennzeichnet, dass** die Verbindung(en) der Formel (I) und/oder ihr(e) Additionssalz(e) mit einer Säure 0,0005 bis 12 Gew.-% des Gesamtgewichts der Farbmittelzusammensetzung ausmachen.

6. Zusammensetzung nach Anspruch 5, **dadurch gekennzeichnet, dass** die Verbindung(en) der Formel (I) und/oder ihr(e) Additionssalz(e) mit einer Säure 0,005 bis 6 Gew.-% des Gesamtgewichts der Farbmittelzusammensetzung ausmachen.

7. Zusammensetzung nach einem der vorhergehenden Ansprüche, **dadurch gekennzeichnet, dass** sie mindestens eine Oxidationsbase enthält, die unter den p-Phenylendiaminen, Bisphenylalkylendiaminen, p-Aminophenolen, o-Aminophenolen und den heterocyclischen Basen ausgewählt ist.

8. Zusammensetzung nach Anspruch 7, **dadurch gekennzeichnet, dass** die Oxidationsbase(n) 0,0005 bis 12 Gew.-% des Gesamtgewichts der Farbmittelzusammensetzung ausmachen.

9. Zusammensetzung nach Anspruch 8, **dadurch gekennzeichnet, dass** die Oxidationsbase(n) 0,005 bis 6 Gew.-% des Gesamtgewichts der Farbmittelzusammensetzung ausmachen.

10. Zusammensetzung nach einem der vorhergehenden Ansprüche, **dadurch gekennzeichnet, dass** sie einen oder mehrere zusätzliche Kuppler enthält, die unter den m-Phenylendiaminen, m-Aminophenolen, m-Dihydroxybenzolen und den heterocyclischen Kupplern ausgewählt sind.

11. Zusammensetzung nach Anspruch 10, **dadurch gekennzeichnet, dass** der oder die zusätzliche(n) Kuppler 0,0001 bis 10 Gew.-% des Gesamtgewichts der Farbmittelzusammensetzung ausmachen.

12. Zusammensetzung nach Anspruch 11, **dadurch gekennzeichnet, dass** der oder die zusätzliche(n) Kuppler 0,005 bis 5 Gew.-% des Gesamtgewichts der Farbmittelzusammensetzung ausmachen.

13. Zusammensetzung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

14. Zusammensetzung nach einem der vorhergehenden Ansprüche, **dadurch gekennzeichnet, dass** zum Färben geeignete Medium (oder der Träger) aus Wasser oder einem Gemisch von Wasser und mindestens einem organischen Lösungsmittel besteht.

15. Zusammensetzung nach einem der vorhergehenden Ansprüche, **dadurch gekennzeichnet, dass** sie einen pH-Wert von 3 bis 12 aufweist.

16. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 15 aufgetragen und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

17. Verfahren nach Anspruch 16, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, und Enzymen, wie Peroxidasen und Oxidoreduktasen, die 2 Elektronen übertragen, ausgewählt ist.

18. Vorrichtung mit mehreren Abteilungen oder "Kit" zum Färben mit mehreren Abteilungen, wobei eine Abteilung eine Farbmittelzusammensetzung nach einem der Ansprüche 1 bis 15 und eine zweite Abteilung eine oxidierende Zusammensetzung enthält.

19. Verbindungen der Formel (I') und deren Additionssalze mit einer Säure: 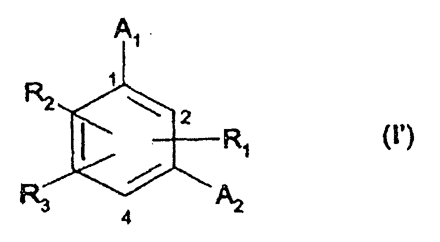 worin bedeuten:
· die Gruppen R₁, R₂ und R₃, die identisch oder voneinander verschieden sein können, ein Wasserstoffatom; ein Halogenatom; Alkyl(C₁₋₆)carbonyl; Aminoalkyl(C₁₋₆)carbonyl; N-Alkyl(C₁₋₆)-aminoalkyl(C₁₋₆)carbonyl; N,N-Dialkyl(C₁₋₆)aminoalkyl(C₁₋₆)-carbonyl; Aminoalkyl(C₁₋₆)carbonylalkyl(C₁₋₆); N-Alkyl(C₁₋₆)-aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl(C₁₋₆)sulfonyl; Aminosulfonyl; N-Alkyl(C₁₋₆)aminosulfonyl; N,N-Dialkyl(C₁₋₆)aminosulfonyl; Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); C₁₋₆-Alkyl; C₁₋₆-Monohydroxyalkyl; C₂₋₆-Polyhydroxyalkyl; Alkoxy(C₁₋₆)-alkyl(C₁₋₆); C₁₋₆-Trifluoralkyl; Cyano; OR₆; oder eine Aminogruppe, die mit Alkyl(C₁₋₆)carbonyl, Alkyl(C₁₋₆)carboxy, Trifluoralkyl(C₁₋₆)carbonyl, Aminoalkyl(C₁₋₆)carbonyl, N-Alkyl(C₁₋₆)aminoalkyl(C₁₋₆)carbonyl, N,N-Dialkyl(C₁₋₆)aminoalkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Alkyl(C₁₋₆)sulfonyl, Aminosulfonyl, N-Alkyl(C₁₋₆)aminosulfonyl, N,N-Dialkyl(C₁₋₆)aminosulfonyl, Thiocarbamoyl oder Formyl geschützt ist;
R₆ C₁₋₆-Alkyl; C₁₋₆-Monohydroxyalkyl; C₂₋₆-Polyhydroxyalkyl; eine Gruppe Z; Alkoxy(C₁₋₆)alkyl(C₁₋₆); Aryl; Benzyl; Carboxyalkyl-(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)-carbamoylalkyl(C₁₋₆); C₁₋₆-Trifluoralkyl; Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)carbonylalkyl(C₁₋₆); Aminoalkyl(C₁₋₆); C₁₋₆-Aminoalkyl, wobei die Aminogruppe mit einer oder zwei Gruppen substituiert ist, die gleich oder verschieden sind und unter den folgenden Gruppen ausgewählt sind: C₁₋₆-Alkyl, C₁₋₆-Monohydroxyalkyl, C₂₋₆-Polyhydroxyalkyl, Alkyl(C₁₋₆)carbonyl, Formyl, Trifluoralkyl(C₁₋₆)carbonyl, Alkyl(C₁₋₆)carboxy, Carbamoyl, N-Alkyl(C₁₋₆)carbamoyl, N,N-Dialkyl(C₁₋₆)carbamoyl, Thiocarbamoyl, Alkyl(C₁₋₆)sulfonyl;
· A1 -NR₄R₅ oder Hydroxy;
· A2 -NR'₄R'₅ oder Hydroxy;
· die Gruppen R₄, R'₄, R₅ und R'₅, die gleich oder voneinander verschieden sind, Wasserstoff; C₁₋₆-Alkyl; C₁₋₆-Monohydroxyalkyl; C₂₋₆-Polyhydroxyalkyl; Alkoxy(C₁₋₆)alkyl(C₁₋₆); Aryl; Benzyl; Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl; Thiocarbamoylalkyl(C₁₋₆); C₁₋₆-Trifluoralkyl; C₁₋₆-Sulfoalkyl; Alkyl(C₁₋₆)carboxyalkyl(C₁₋₆); Alkyl(C₁₋₆)sulfinylalkyl(C₁₋₆); Aminosulfonylalkyl(C₁₋₆); N-Alkyl(C₁₋₆)aminosulfonylalkyl(C₁₋₆); N,N-Dialkyl(C₁₋₆)aminosulfonylalkyl(C₁₋₆); Alkyl(C₁₋₆)carbonylalkyl(C₁₋₆); Aminoalkyl(C₁₋₆); C₁₋₆-Aminoalkyl, wobei die Aminogruppe mit einer oder zwei gleichen oder verschiedenen Gruppen substituiert ist, die unter Alkyl, C₁₋₆-Monohydroxyalkyl, C₂₋₆-Polyhydroxyalkyl, Alkyl(C₁₋₆)-carbonyl, Carbamoyl, N-Alkyl(C₁₋₆)carbamoyl oder N,N-Dialkyl(C₁₋₆)carbamoyl, Alkyl(C₁₋₆)sulfonyl, Formyl, Trifluoralkyl(C₁₋₆) carbonyl, Alkyl(C₁₋₆)carboxy oder Thiocarbamoyl ausgewählt sind;
Z eine Gruppe der folgenden Formel (II) 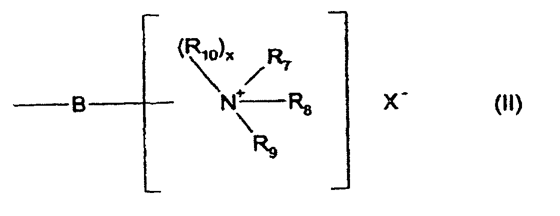 worin bedeuten:
- B eine Verbindungsgruppe, die eine Alkylkette bedeutet, welche vorzugsweise 1 bis 14 Kohlenstoffatome aufweist und die geradkettig oder verzweigt vorliegt;
- die Gruppen R₇, R₈ und R₉, die gleich oder verschieden sind, C₁₋₆-Alkyl; C₁₋₆-Monohydroxyalkyl; C₂₋₆-Polyhydroxyalkyl; Alkoxy(C₁₋₆)alkyl(C₁₋₆); Cyanoalkyl(C₁₋₆); Aryl; Benzyl; Carbamoylalkyl(C₁₋₆); Trialkyl(C₁₋₆)silanalkyl(C₁₋₆) oder eine C₁₋₆-Aminogruppe, deren Aminogruppe mit Alkyl(C₁₋₆)carbonyl, Carbamoyl oder Alkyl(C₁₋₆)sulfonyl geschützt ist, wobei zwei der Gruppen R₇, R₈ und R₉ auch gemeinsam mit dem Stickstoffatom, an das sie gebunden sind, einen 5- oder 6-gliedrigen gesättigten Ring auf Kohlenstoffbasis oder Ring mit einem oder mehreren Heteroatomen bilden können, wobei der Ring gegebenenfalls substituiert ist mit Halogen, Hydroxy, C₁₋₆-Alkyl, C₁₋₆-Monohydroxyalkyl, C₂₋₆-Polyhydroxyalkyl, Nitro, Cyano, Cyanoalkyl(C₁₋₆), C₁₋₆-Alkoxy, Trialkyl(C₁₋₆)silanalkyl(C₁₋₆), Amido, Aldehyde, Carboxy, Alkylcarbonyl(C₁₋₆), Thio, C₁₋₆-Thioalkyl, Alkyl(C₁₋₆)thio, Amino, einer Aminogruppe, die mit Alkyl(C₁₋₆)carbonyl, Carbamoyl oder Alkyl(C₁₋₆)-sulfonyl geschützt ist;
- X⁻ ein einwertiges oder zweiwertiges Anion, das vorzugsweise unter den Halogenatmen, wie beispielsweise Chlor, Brom, Fluor oder Iod, Hydroxid, Hydrogensulfat und Alkyl(C₁₋₆)-sulfaten, beispielsweise Methyl- oder Ethylsulfat, ausgewählt ist;
- R₁₀ C₁₋₆-Alkyl; C₁₋₆-Monohydroxyalkyl; C₂₋₆-Polyhydroxyalkyl; Aryl; Benzyl; Aminoalkyl(C₁₋₆) oder eine C₁₋₆-Aminoalkylgruppe, deren Aminogruppe mit Alkyl(C₁₋₆)carbonyl, Carbamoyl oder Alkyl(C₁₋₆)sulfonyl geschützt ist; Carboxyalkyl(C₁₋₆); Cyanoalkyl(C₁₋₆); Carbamoylalkyl(C₁₋₆); C₁₋₆-Trifluoralkyl; Trialkyl(C₁₋₆)silanalkyl(C₁₋₆); Sulfonamidoalkyl(C₁₋₆); Alkyl(C₁₋₆)-carboxyalkyl(C₁₋₆); Alkyl(C₁₋₆)sulfinylalkyl(C₁₋₆); Alkyl(C₁₋₆)-sulfonylalkyl(C₁₋₆); Alkyl(C₁₋₆)ketoalkyl(C₁₋₆); N-Alkyl(C₁₋₆)-carbamoylalkyl; N-Alkyl(C₁₋₆)sulfonamidosulfonylalkyl(C₁₋₆);
- x 0 oder 1, mit den folgenden Maßgaben:
- wenn x = 0, ist die Verbindungsgruppe B an das Stickstoffatom gebunden, das die Gruppen R₇ bis R₉ trägt,
- wenn x = 1, bilden zwei der Gruppen R₇ bis R₉ gemeinsam mit dem Stickstoffatom, an das sie gebunden sind, einen oben definierten gesättigten, 5- oder 6-gliedrigen Ring und die Verbindungsgruppe B wird von einem Kohlenstoffatom des gesättigten Rings getragen;
mit der Maßgabe, dass die Anzahl der Gruppen Z 1 beträgt.

20. Zusammensetzung nach Anspruch 19, **dadurch gekennzeichnet, dass** die Verbindungen der Formel (I') ausgewählt sind unter:
- [2-(2,4-Diaminophenoxy)-ethyl]-diethyl-methylammoniumchlorid;
- 1-[3-(2,4-Diaminophenoxy)-propyl]-1,4-dimethyl-piperazin-1-ium-chlorid;
- [3-(2,4-Diaminophenoxy)-propyl]-triethyl-ammoniumchlorid;
- [2-(4-Amino-2-hydroxy-phenoxy)-ethyl]-diethyl-methylammoniumchlorid;
- 1-[3-(2-Amino-4-methylamino-phenoxy)-propyl]-1-methylpiperidiniumchlorid;
- [2-(2,4-Diaminophenoxy)-ethyl]-diethyl-methylammoniumchlorid;
- 1-[3-(2,4-Diaminophenoxy)-propyl]-1-methylpyrrolidiniumchlorid;
- und deren Additionssalzen mit einer Säure.

21. Verwendung von Verbindungen der Formel (I), wie sie in einem der Ansprüche 1 bis 4 definiert wurden, als Kuppler für das oxidative Färben von Keratinfasern, insbesondere menschlichen Keratinfasern, wie Haaren.

## Claims

1. Composition for the oxidation dyeing of keratin fibres, and in particular of human keratin fibres such as the hair, **characterized in that** it comprises, in a medium which is suitable for dyeing, at least one monobenzenic coupler of formula (I) below, and/or at least one of the addition salts thereof with an acid: 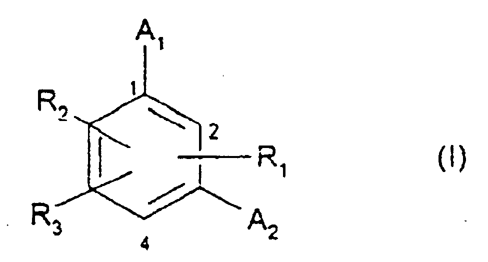 in which:
• R₁, R₂ and R₃, which may be identical or different, represent a hydrogen atom; a halogen atom; a (C₁-C₆)alkylcarbonyl radical; an amino (C₁-C₆)alkylcarbonyl radical; an N- (C₁-C₆)alkylamino-(C₁-C₆)alkylcarbonyl radical; an N,N-di(C₁-C₆)-alkylamino(C₁-C₆)alkylcarbonyl radical; an amino(C₁-C₆)alkylcarbonyl(C₁-C₆)alkyl radical; an N-(C₁-C₆)alkylamino (C₁-C₆)alkylcarbonyl (C₁-C₆)alkyl radical; an N,N-di(C₁-C₆)alkylamino(C₁-C₆)alkylcarbonyl(C₁-C₆)alkyl radical; a carboxyl radical; a (C₁-C₆)alkylcarboxyl radical; a C₁-C₆ alkylsulphonyl radical; an aminosulphonyl radical; a C₁-C₆ N-alkylaminosulphonyl radical; an N,N-di(C₁-C₆)alkylaminosulphonyl radical; a C₁-C₆ aminosulphonylalkyl radical; an N-(C₁-C₆)alkylaminosulphonyl(C₁-C₆)alkyl radical; an N, N-di (C₁-C₆)alkylaminosulphonyl (C₁-C₆)alkyl radical; a carbamyl radical; an N-(C₁-C₆)alkylcarbamyl radical; an N,N-di(C₁-C₆)alkylcarbamyl radical; a carbamyl (C₁-C₆)alkyl radical; an N-(C₁-C₆)alkylcarbamyl(C₁-C₆)alkyl radical; an N, N-di (C₁-C₆)alkylcarbamyl (C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; a group OR₆; or an amino group protected with a (C₁-C₆)alkylcarbonyl, (C₁-C₆)alkylcarboxyl, trifluoro (C₁-C₆)alkylcarbonyl, amino (C₁-C₆)alkylcarbonyl, N-(C₁-C₆)alkylamino(C₁-C₆)alkylcarbonyl, N, N-di (C₁-C₆)alkylamino(C₁-C₆)alkylcarbonyl, (C₁-C₆)alkylcarboxyl, carbamyl, N-(C₁-C₆)alkylcarbamyl, N,N-di(C₁-C₆)alkylcarbamyl, C₁-C₆ alkylsulphonyl, aminosulphonyl, C₁-C₆ N-alkylaminosulphonyl, N,N-di(C₁-C₆)alkylaminosulphonyl, thiocarbamyl or formyl radical;
• R₆ denotes a C₁-C₆ alkyl radical; a C₁-C₆ monohydroxyalkyl radical; a C₂-C₆ polyhydroxyalkyl radical; a group Z; a (C₁-C₆)alkoxy(C₁-C₆)alkyl radical; an aryl radical; a benzyl radical; a carboxy(C₁-C₆)alkyl radical; a (C₁-C₆)alkylcarboxy-(C₁-C₆)alkyl radical; a cyano(C₁-C₆)alkyl radical; a carbamyl (C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ trifluoroalkyl radical; a C₁-C₆ aminosulphonylalkyl radical; an N-(C₁-C₆)alkylaminosulphonyl(C₁-C₆)alkyl radical; an N,N-di(C₁-C₆)alkylaminosulphonyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro(C₁-C₆)alkylcarbonyl, (C₁-C₆)alkylcarboxyl, carbamyl, N-(C₁-C₆)alkylcarbamyl, N,N-di-(C₁-C₆)alkylcarbamyl, thiocarbamyl and C₁-C₆ alkylsulphonyl radicals;
• A1 represents a group -NR₄R₅ or a hydroxyl radical;
• A2 represents a group -NR'₄R'₅ or a hydroxyl radical;
• R₄, R'₄, R₅ and R'₅, which may be identical or different, represent a hydrogen atom; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a cyano(C₁-C₆)alkyl radical; a carbamyl(C₁-C₆)alkyl radical; an N-(C₁-C₆)alkylcarbamyl (C₁-C₆)alkyl radical; an N,N-di(C₁-C₆)alkylcarbamyl(C₁-C₆)alkyl radical; a thiocarbamyl (C₁-C₆)alkyl radical; a C₁-C₆ trifluoroalkyl radical; a C₁-C₆ sulphoalkyl radical; a (C₁-C₆)alkylcarboxy(C₁-C₆)alkyl radical; a (C₁-C₆)alkylsulphinyl(C₁-C₆)alkyl radical; a C₁-C₆ aminosulphonylalkyl radical; an N-(C₁-C₆)alkylaminosulphonyl(C₁-C₆)alkyl radical; an N, N-di (C₁-C₆)alkylaminosulphonyl (C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, carbamyl, N-(C₁-C₆)alkylcarbamyl or N,N-di(C₁-C₆)-alkylcarbamyl, C₁-C₆ alkylsulphonyl, formyl, trifluoro(C₁-C₆)alkylcarbonyl, (C₁-C₆)alkylcarboxyl and thiocarbamyl radicals;
• Z represents a group of formula (II) below: 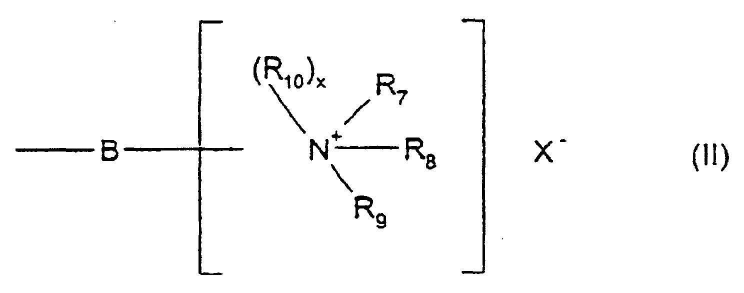 in which:
• B is a linker arm which represents a linear or branched alkyl chain preferably containing from 1 to 14 carbon atoms;
• R₇, R₈ and R₉, which may be identical or different, represent a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a cyano(C₁-C₆)alkyl radical; an aryl radical; a benzyl radical; a carbamyl(C₁-C₆)alkyl radical; a tri(C₁-C₆)alkylsilane(C₁-C₆)alkyl radical or a C₁-C₆ aminoalkyl radical in which the amine is protected with a (C₁-C₆)alkylcarbonyl, carbamyl or (C₁-C₆)alkylsulphonyl radical; two of the radicals R₇, R₈ and R₉ can also form, together with the nitrogen atom to which they are attached, a 5- or 6-membered saturated carbon-based ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehydo radical, a carboxyl radical, a (C₁-C₆)alkylcarbonyl radical, a thio radical, a C₁-C₆ thioalkyl radical, a (C₁-C₆)alkylthio radical, an amino radical, an amino radical protected with a (C₁-C₆)alkylcarbonyl, carbamyl or (C₁-C₆)alkylsulphonyl radical;
• X⁻ represents a monovalent or divalent anion;
• R₁₀ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy(C₁-C₆)alkyl radical; a cyano(C₁-C₆)alkyl radical; a carbamyl(C₁-C₆)alkyl radical; a C₁-C₆ trifluoroalkyl radical; a tri (C₁-C₆)alkylsilane(C₁-C₆)alkyl radical; a C₁-C₆ sulphonamidoalkyl radical; a (C₁-C₆)alkylcarboxy(C₁-C₆)alkyl radical; a (C₁-C₆)alkylsulphinyl(C₁-C₆)alkyl radical; a (C₁-C₆)alkylsulphonyl (C₁-C₆)alkyl radical; a (C₁-C₆)alkylketo(C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• x is an integer equal to 0 or 1; with the following conditions:
- when x = 0, then the linker arm B is attached to the nitrogen atom bearing the radicals R₇ to R₉;
- when x = 1, then two of the radicals R₇ to R₉ form, together with the nitrogen atom to which they are attached, a saturated 5- or 6-membered ring as defined above, and the linker arm B is borne by a carbon atom of the said saturated ring;
it being understood that the number of groups Z is equal to 1.

2. Composition according to Claim 1, **characterized in that** X⁻ represents a halogen atom such as chlorine, bromine, fluorine or iodine, a hydroxide, a hydrogen sulphate or a (C₁-C₆)alkyl sulphate.

3. Composition according to Claim 1 or 2, **characterized in that** the compounds of formula (I) are chosen from:
- [2-(2,4-diaminophenoxy)ethyl]diethylmethylammonium chloride;
- 1-[3-(2,4-diaminophenoxy)propyl]-1,4-dimethylpiperazin-1-ium chloride;
- [3-(2,4-diaminophenoxy)propyl]triethylammonium chloride;
- [2-(4-amino-2-hydroxyphenoxy)ethyl]diethylmethylammonium chloride;
- 1-[3-(2-amino-4-methylaminophenoxy)propyl]-1-methylpiperidinium chloride;
- [2-(2,4-diaminophenoxy)ethyl]diethylmethylammonium chloride;
- 1-[3-(2,4-diaminophenoxy)propyl]-1-methylpyrrolidinium chloride;
and the addition salts thereof with an acid.

4. Composition according to Claim 3, chracterized in that the compounds of formula (I) are chosen from:
- [2-(2,4-diaminophenoxy)ethyl]diethylmethylammonium chloride;
- 1-[3-(2,4-diaminophenoxy)propyl]-1,4-dimethylpiperazin-1-ium chloride;
and the addition salts thereof with an acid.

5. Composition according to any one of the preceding claims, **characterized in that** the compound(s) of formula (I) and/or the addition salt(s) thereof with an acid represent(s) from 0.0005% to 12% by weight relative to the total weight of the dye composition.

6. Composition according to Claim 5, **characterized in that** the compound(s) of formula (I) and/or the addition salt(s) thereof with an acid represent(s) from 0.005% to 6% by weight relative to the total weight of the dye composition.

7. Composition according to any one of the preceding claims, **characterized in that** it contains at least one oxidation base chosen from para-phenylenediamines, bis(phenyl)alkylenediamines, para-aminophenols, ortho-aminophenols and heterocyclic bases.

8. Composition according to Claim 7, **characterized in that** the oxidation base(s) represent(s) from 0.0005% to 12% by weight relative to the total weight of the dye composition.

9. Composition according to Claim 8, **characterized in that** the oxidation base(s) represent(s) from 0.005% to 6% by weight relative to the total weight of the dye composition.

10. Composition according to any one of the preceding claims, **characterized in that** it contains one or more additional couplers chosen from meta-phenylenediamines, meta-aminophenols, meta-diphenols and heterocyclic couplers.

11. Composition according to Claim 10, **characterized in that** the additional coupler(s) represent(s) from 0.0001% to 10% by weight relative to the total weight of the dye composition.

12. Composition according to Claim 11, **characterized in that** the additional coupler(s) represent(s) from 0.005% to 5% by weight relative to the total weight of the dye composition.

13. Composition according to any one of the preceding claims, **characterized in that** the addition salts with an acid are chosen from the hydrochlorides, hydrobromides, sulphates, citrates, succinates, tartrates, lactates and acetates.

14. Composition according to any one of the preceding claims, **characterized in that** the medium which is suitable for dyeing (or support) consists of water or a mixture of water and at least one organic solvent.

15. Composition according to any one of the preceding claims, **characterized in that** it has a pH of between 3 and 12.

16. Process for the oxidation dyeing of keratin fibres, and in particular of human keratin fibres such as the hair, **characterized in that** at least one dye composition as defined in any one of Claims 1 to 15 is applied to the said fibres, and **in that** the colour is developed at acidic, neutral or alkaline pH using an oxidizing agent which is added, just at the time of use, to the dye composition, or which is present in an oxidizing composition that is applied simultaneously or sequentially.

17. Process according to Claim 16, **characterized in that** the oxidizing agent is chosen from hydrogen peroxide, urea peroxide, alkali metal bromates, persalts such as perborates and persulphates, and enzymes such as peroxidases and 2-electron oxidoreductases.

18. Multi-compartment dyeing device or multi-compartment dyeing kit, a first compartment of which contains a dye composition as defined in any one of Claims 1 to 15 and a second compartment of which contains an oxidizing composition.

19. Compounds of formula (I'), and the addition salts thereof with an acid: 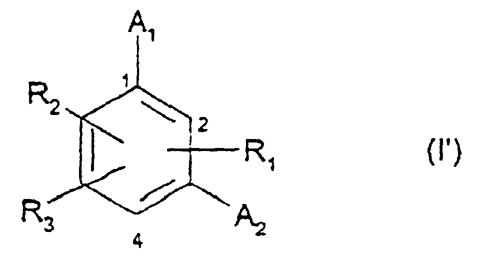 in which
• R₁, R₂ and R₃, which may be identical or different, represent a hydrogen atom; a halogen atom; a (C₁-C₆)alkylcarbonyl radical; an amino(C₁-C₆)alkylcarbonyl radical; an N- (C₁-C₆) alkylamino (C₁-C₆)-alkylcarbonyl radical; an N,N-di(C₁-C₆)alkylamino(C₁-C₆)alkylcarbonyl radical; an amino (C₁-C₆)alkylcarbonyl (C₁-C₆)alkyl radical; an N- (C₁-C₆)alkylamino(C₁-C₆) alkylcarbonyl(C₁-C₆) alkyl radical; an N,N-di(C₁-C₆)alkylamino(C₁-C₆)alkylcarbonyl(C₁-C₆)alkyl radical; a carboxyl radical; a (C₁-C₆)alkylcarboxyl radical; a C₁-C₆ alkylsulphonyl radical; an aminosulphonyl radical; a C₁-C₆ N-alkylaminosulphonyl radical; an N,N-di(C₁-C₆)alkylaminosulphonyl radical; a C₁-C₆ aminosulphonylalkyl radical; an N-(C₁-C₆)alkylaminosulphonyl(C₁-C₆)alkyl radical; an N, N-di (C₁-C₆) alkylaminosulphonyl (C₁-C₆)alkyl radical; a carbamyl radical; an N-(C₁-C₆)alkylcarbamyl radical; an N,N-di(C₁-C₆)alkylcarbamyl radical; a carbamyl(C₁-C₆)alkyl radical; an N- (C₁-C₆)alkylcarbamyl (C₁-C₆) alkyl radical; an N, N-di (C₁-C₆)alkylcarbamyl(C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; a group OR₆; or an amino group protected with a (C₁-C₆)alkylcarbonyl, (C₁-C₆)alkylcarboxyl, trifluoro(C₁-C₆) alkylcarbonyl, amino(C₁-C₆)alkylcarbonyl, N-(C₁-C₆)alkylamino(C₁-C₆)alkylcarbonyl, N, N-di (C₁-C₆)alkylamino(C₁-C₆)alkylcarbonyl, (C₁-C₆)alkylcarboxyl, carbamyl, N-(C₁-C₆)alkylcarbamyl, N,N-di(C₁-C₆)alkylcarbamyl, C₁-C₆ alkylsulphonyl, aminosulphonyl, C₁-C₆ N-alkylaminosulphonyl, N,N-di(C₁-C₆)alkylaminosulphonyl, thiocarbamyl or formyl radical;
• R₆ denotes a C₁-C₆ alkyl radical; a C₁-C₆ monohydroxyalkyl radical; a C₂-C₆ polyhydroxyalkyl radical; a group Z; a (C₁-C₆)alkoxy(C₁-C₆)alkyl radical; an aryl radical; a benzyl radical; a carboxy(C₁-C₆)alkyl radical; a (C₁-C₆)alkylcarboxy-(C₁-C₆)alkyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a C₁-C₆ trifluoroalkyl radical; a C₁-C₆ aminosulphonylalkyl radical; an N-(C₁-C₆)alkylaminosulphonyl(C₁-C₆)alkyl radical; an N, N-di (C₁-C₆)alkylaminosulphonyl (C₁-C₆)alkyl radical; a (C₁-C₆)alkylsulphinyl(C₁-C₆)alkyl radical; a (C₁-C₆)alkylsulphonyl (C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, formyl, trifluoro(C₁-C₆)alkylcarbonyl, (C₁-C₆)alkylcarboxyl, carbamyl, N-(C₁-C₆)alkylcarbamyl, N,N-di(C₁-C₆)alkylcarbamyl, thiocarbamyl and C₁-C₆ alkylsulphonyl radicals;
• A1 represents a group -NR₄R₅ or a hydroxyl radical;
• A2 represents a group -NR'₄R'₅ or a hydroxyl radical;
• R₄, R'₄, R₅ and R'₅, which may be identical or different, represent a hydrogen atom; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; an aryl radical; a benzyl radical; a cyano(C₁-C₆)alkyl radical; a carbamyl(C₁-C₆)alkyl radical; an N-(C₁-C₆)alkylcarbamyl(C₁-C₆)alkyl radical; an N,N-di(C₁-C₆)alkylcarbamyl(C₁-C₆)alkyl radical; a thiocarbamyl(C₁-C₆)alkyl radical; a C₁-C₆ trifluoroalkyl radical; a C₁-C₆ sulphoalkyl radical; a (C₁-C₆)alkylcarboxy(C₁-C₆)alkyl radical; a (C₁-C₆)alkylsulphinyl(C₁-C₆)alkyl radical; a C₁-C₆ aminosulphonylalkyl radical; an N-(C₁-C₆)alkylaminosulphonyl(C₁-C₆)alkyl radical; an N, N-di (C₁-C₆)alkylaminosulphonyl(C₁-C₆)alkyl radical; a (C₁-C₆)alkylcarbonyl(C₁-C₆)alkyl radical; a C₁-C₆ aminoalkyl radical; a C₁-C₆ aminoalkyl radical in which the amine is substituted with one or two identical or different radicals chosen from C₁-C₆ alkyl, C₁-C₆ monohydroxyalkyl, C₂-C₆ polyhydroxyalkyl, (C₁-C₆)alkylcarbonyl, carbamyl, N-(C₁-C₆)alkylcarbamyl or N, N-di (C₁-C₆)alkylcarbamyl, C₁-C₆ alkylsulphonyl, formyl, trifluoro(C₁-C₆)alkylcarbonyl, (C₁-C₆)alkylcarboxyl and thiocarbamyl radicals;
• Z represents a group of formula (II) below: 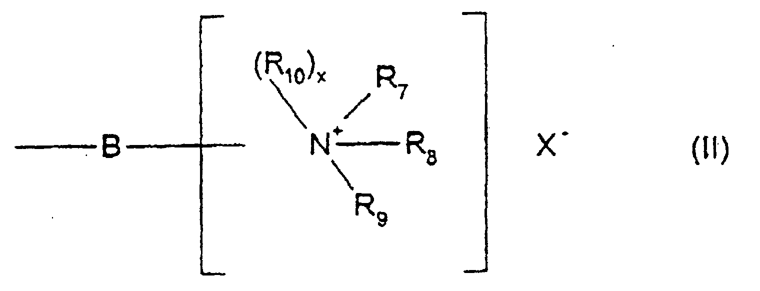 in which:
• B is a linker arm which represents a linear or branched alkyl chain preferably containing from 1 to 14 carbon atoms;
• R₇, R₈ and R₉, which may be identical or different, represent a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a cyano(C₁-C₆)alkyl radical; an aryl radical; a benzyl radical; a carbamyl(C₁-C₆)alkyl radical; a tri(C₁-C₆)alkylsilane(C₁-C₆)alkyl radical or a C₁-C₆ aminoalkyl radical in which the amine is protected with a (C₁-C₆)alkylcarbonyl, carbamyl or (C₁-C₆)alkylsulphonyl radical; two of the radicals R₇, R₈ and R₉ can also form, together with the nitrogen atom to which they are attached, a 5- or 6-membered saturated carbon-based ring or a ring containing one or more hetero atoms, it being possible for the said ring to be unsubstituted or substituted with a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a cyano(C₁-C₆)alkyl radical, a C₁-C₆ alkoxy radical, a tri(C₁-C₆)alkylsilane(C₁-C₆)alkyl radical, an amido radical, an aldehyde radical, a carboxyl radical, a (C₁-C₆)alkylcarbonyl radical, a thio radical, a C₁-C₆ thioalkyl radical, a (C₁-C₆)alkylthio radical, an amino radical, an amino radical protected with a (C₁-C₆)alkylcarbonyl, carbamyl or (C₁-C₆)alkylsulphonyl radical;
• X⁻ represents a monovalent or divalent anion and is preferably chosen from a halogen atom such as chlorine, bromine, fluorine or iodine, a hydroxide, a hydrogen sulphate or a (C₁-C₆)alkyl sulphate such as, for example, a methyl sulphate or an ethyl sulphate;
• R₁₀ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical, a C₁-C₆ aminoalkyl radical in which the amine is protected with a (C₁-C₆)alkylcarbonyl, carbamyl or C₁-C₆ alkylsulphonyl radical; a carboxy(C₁-C₆)alkyl radical; a cyano(C₁-C₆)alkyl radical; a carbamyl(C₁-C₆)alkyl radical; a C₁-C₆ trifluoroalkyl radical; a tri(C₁-C₆)alkylsilane(C₁-C₆)alkyl radical; a C₁-C₆ sulphonamidoalkyl radical; a (C₁-C₆)alkylcarboxy(C₁-C₆)alkyl radical; a (C₁-C₆)alkylsulphinyl (C₁-C₆)alkyl radical; a (C₁-C₆)alkylsulphonyl (C₁-C₆)alkyl radical; a (C₁-C₆)alkylketo(C₁-C₆)alkyl radical; an N-(C₁-C₆)alkylcarbamyl(C₁-C₆)alkyl radical; an N-(C₁-C₆)alkylsulphonamido(C₁-C₆)alkyl radical;
• x is an integer equal to 0 or 1; with the following conditions:
- when x = 0, then the linker arm B is attached to the nitrogen atom bearing the radicals R₇ to R_{9;}
- when x = 1, then two of the radicals R₇ to R₉ form, together with the nitrogen atom to which they are attached, a saturated 5- or 6-membered ring as defined above, and the linker arm B is borne by a carbon atom of the said saturated ring;
it being understood that the number of groups Z is equal to 1.

20. Compounds according to Claim 19, **characterized in that** they are chosen from:
- [2-(2,4-diaminophenoxy)ethyl]diethylmethylammonium chloride;
- 1-[3-(2,4-diaminophenoxy)propyl]-1,4-dimethylpiperazin-1-ium chloride;
- [3-(2,4-diaminophenoxy)propyl]triethylammonium chloride;
- [2-(4-amino-2-hydroxyphenoxy)ethyl]diethylmethylammonium chloride;
- 1-[3-(2-amino-4-methylaminophenoxy)propyl]-1-methylpiperidinium chloride;
- [2-(2,4-diaminophenoxy)ethyl]diethylmethylammonium chloride;
- 1-[3-(2,4-diaminophenoxy)propyl]-1-methylpyrrolidinium chloride;
and the addition salts thereof with an acid.

21. Use of the compounds of formula (I) as defined in any one of Claims 1 to 4 as couplers for the oxidation dyeing of keratin fibres, and in particular of human keratin fibres such as the hair.
